# FASCICULE DE BREVET EUROPEEN

(11) **EP 2 080 436 B1**
(45) Date de publication et mention de la délivrance du brevet: **07.05.2014**
(21) Numéro de dépôt: 09305035.9
(22) Date de dépôt: 14.01.2009
(51) Int. Cl.: A23L 1/30, A61K 36/85, A61K 8/97, A23F 3/16, A23F 3/22, A23L 1/025

(54) **Procédé de préparation d'extraits végétaux permettant l'obtention d'une nouvelle forme galénique**
Verfahren zur Herstellung von Pflanzenextrakten, die den Erhalt einer neuen galenischen Formel ermöglichen
Method for preparing plant extracts allowing a new dosage form to be obtained

(30) Priorité: 14.01.2008 FR 0850185
(43) Date de publication de la demande: 22.07.2009
(73) Titulaire: Thiomed, 03800 Saint Bonnet de Rochefort (FR)
(72) Inventeur: Dubourdeaux, Michel, 03140 Taxat (FR)
(74) Mandataire: Denjean, Eric

(56) Documents cités:
- EP-A- 0 294 177
- WO-A-2006/078699
- FR-A- 2 754 448
- FR-A- 2 865 652
- FR-A- 2 892 933
- ZA-A- 200 400 776
- DATABASE CAPLUS [Online] CHEMICAL ABSTRACTS SERVICE, COLUMBUS, OHIO, US; XP002497440 extrait de STN Database accession no. 2007:317258 & CN 1 927 013 A (FUJIAN AGRICULTURE AND FORESTRY UNIVERSITY) 14 mars 2007 (2007-03-14)
- DATABASE CAPLUS [Online] CHEMICAL ABSTRACTS SERVICE, COLUMBUS, OHIO, US; XP002497441 extrait de STN Database accession no. 2005:1238239 & CN 1 589 847 A (SHANGHAI INSTITUTE OF DOMESTIC ANIMAL PARASITOLOGY) 9 mars 2005 (2005-03-09)
- DATABASE CAPLUS [Online] CHEMICAL ABSTRACTS SERVICE, COLUMBUS, OHIO, US; XP002497442 extrait de STN Database accession no. 2004:1105107 & KR 200 125 274 A (GENOMINE INC) 6 avril 2001 (2001-04-06)
- DATABASE CAPLUS [Online] CHEMICAL ABSTRACTS SERVICE, COLUMBUS, OHIO, US; XP002497443 extrait de STN Database accession no. 2006:1116222 & CN 1 712 402 A (SERICULTURE AND FARM PRODUCT PROCESSING RESEARCH INSTITUTE) 28 décembre 2005 (2005-12-28)
- DATABASE CAPLUS [Online] CHEMICAL ABSTRACTS SERVICE, COLUMBUS, OHIO, US; XP002497444 extrait de STN Database accession no. 2006:901055 & CN 1 824 084 A (PEOP. REP. CHINA) 3 août 2006 (2006-08-03)
- WANG ET AL.: "Recent advances in extraction of nutraceuticals from plants" TRENDS IN FOOD SCIENCE & TECHNOLOGY, vol. 17, 2006, pages 300-312, XP002497439

## Description

### Domaine de l'invention

L'invention a pour objet un nouveau procédé de préparation d'extraits végétaux, selon lequel les principes actifs sont imprégnés sur un support de plante.

Il s'avère qu'une telle forme galénique est plus active et plus facilement assimilable. Les extraits ainsi préparés peuvent être utilisés dans des compositions alimentaires, cosmétiques ou pharmaceutiques, pour réaliser des tisanes.

### Etat de la technique

Dans le cadre de l'utilisation des plantes pour leurs vertus thérapeutiques et depuis des millénaires, la forme de présentation la plus communément admise est la forme liquide. Ses avantages sont nombreux, notamment une meilleure biodisponibilité et la possibilité de sa préparation extemporanée. En contrepartie, elle présente des inconvénients, tels qu'un dosage faible en actifs nécessitant des volumes importants, le goût et l'odeur, ainsi que les contraintes liées à sa préparation.

A ce titre, la tisane de plantes est la référence, prescrite ou utilisée en automédication.

Dans le passé, les autres formes galéniques étaient réservées à la prescription et relevaient du domaine de l'apothicaire. Les pilules et les cachets pouvaient contenir des poudres de plantes, leur dosage étant faible et la posologie importante.

Aujourd'hui, les choses ont peu changé dans ce domaine. La notion d'extrait ou de poudre de plante est toujours présente. L'extrait est devenu sec par soustraction de l'eau, mais de ce fait s'éloigne du fondamental de la phytothérapie, à savoir la notion de « totum » qui correspond à l'ensemble des éléments constitutifs de la plante.

Les évolutions ont davantage touché la forme qui est devenue plus moderne, notamment via une prise unique et la praticité d'emploi.

Très récemment, les études du PNNS ont démontré que la prise journalière d'une tisane de plantes comme la verveine ou le tilleul permet de limiter l'effet oxydatif et diminue de moitié l'apparition de certains cancers.

La tisane traditionnelle est donc aujourd'hui la seule forme de préparation phytothérapique permettant de garantir une quantité d'actifs suffisante permettant une efficacité avérée.

La problématique est que la préparation d'une tisane traditionnelle est longue et fastidieuse. En règle générale, il faut effectuer une décoction de 10 à 20 grammes de plantes pendant 15 à 20 minutes dans 1 litre d'eau, à consommer dans la journée. Peu de personnes se plient à ce protocole et la majorité préfère acheter des infusions en sachets, certes plus pratiques mais, bien sûr, bien moins efficaces du fait de leur faible concentration (1,5 à 2 grammes par sachet, avec une préparation qui n'excède pas 3 minutes).

Ainsi, il ressort que l'évolution d'un produit de phytothérapie doit se faire en respectant certaines règles :
- le respect de la notion d'intégralité, qui est un des préceptes de la phytothérapie ;
- le respect de l'intégrité des constituants, en employant une méthode non traumatisante pour le traitement du végétal et qui permet le maintien de ses qualités biologiques ;
- la proposition de techniques innovantes dans le traitement du végétal, permettant de se démarquer des formes classiquement utilisées.

En résumé, la poudre de plante est aujourd'hui la plus communément utilisée dans les formes galéniques de phytothérapie. Elle présente toutefois de nombreux inconvénients : faiblesse des actifs ; quantité relativement importante à absorber pour obtenir un effet physiologique (2 à 3 grammes par jour correspondent à la prise de 7 à 10 gélules de 300 mg) ; faible biodisponibilité ; intégrité non respectée en raison d'un traitement des plantes séchage/broyage souvent drastique.

Comme déjà mentionné, la tisane est la forme la plus efficace et recommandée, lorsqu'elle est réalisée dans les conditions de la pharmacopée. Or, aujourd'hui, les consommatrices se tournent davantage vers les infusions classiques que vers les tisanes thérapeutiques, pour les raisons citées plus haut.

Il est donc nécessaire de concevoir une nouvelle forme galénique qui permette à la fois :
- de s'absoudre des inconvénients majeurs des poudres de plantes médicinales, en augmentant la biodisponibilité des principes actifs de la plante ;
- de réduire la prise et le nombre de gélules, en augmentant la quantité de principes actifs dans la préparation ;
- de pouvoir réaliser, sous forme d'infusions, des tisanes aussi concentrées que les tisanes thérapeutiques, mais avec un temps de préparation d'une infusion classique.

EP 0294177 A décrit un procédé d'extraction par entrainement à la vapeur d'agents aromatiques à partir d'une matière végétale (feuilles de thé), l'extrait aromatique pouvant être ajouté à la matière végétale épuisée après séchage de celle-ci.

La présente invention s'inscrit donc dans la recherche de nouveaux procédés de préparation d'extraits végétaux pour la réalisation d'une tisane respectant l'intégrité et l'intégralité des composants végétaux, et dans la recherche de nouvelles formules galéniques présentant une meilleure biodisponibilité et une plus grande concentration.

### Exposé de l'invention

Ainsi, la présente invention concerne un nouveau procédé de préparation d'extraits végétaux, à partir de matière végétale.

Un tel procédé comprend les étapes essentielles suivantes :
- une étape de contact entre la matière végétale et un solvant ;
- au moins une étape d'extraction des principes actifs ;
- une étape de séchage.

Selon une première caractéristique, le procédé peut être conduit sur de la matière végétale sèche ou fraîche, de même que sur une partie de plante ou une plante entière.

La première étape consiste donc à placer la matière végétale au contact d'un solvant, dans lequel va avoir lieu l'extraction des principes actifs. Ce solvant qui peut être aqueux, hydro-organique ou purement organique, est avantageusement un mélange hydro-alcoolique, comme par exemple un mélange 50/50 (volume) eau/alcool, notamment un mélange hydro-éthanolique à 50/50 V/V. Des exemples de solvants organiques susceptibles d'être utilisés au cours de cette étape sont l'éthanol, le méthanol, l'acétone ou leurs mélanges. Cette étape est assimilable à une phase de macération.

La seconde étape correspond à la phase d'extraction à proprement parler. Une ou plusieurs étapes d'extraction peuvent être mises en oeuvre. Les conditions d'extraction sont déterminées en fonction de la nature de la matière végétale en présence.

De manière avantageuse, cette extraction est réalisée sous hyperfréquence, ou tout autre moyen de chauffage conventionnel, et sous pression réduite, ces deux conditions réunies permettant d'extirper l'ensemble des actifs de la plante et ainsi de respecter la notion d'intégralité, sans modification de leur intégrité.

De manière privilégiée, le générateur micro-onde GMP 60 K qui équipe l'enceinte d'extraction délivre une puissance de 600 à 6000 W dans la bande de fréquence comprise entre 300 MHz et 300 GHz, avantageusement 2450 MHz. L'utilisation des hyperfréquences comme moyen de chauffage, par rapport aux techniques conventionnelles, permet d'écourter la phase d'extraction car le chauffage de la masse végétale est très court.

La notion de pression réduite implique une mise sous vide, au moins partiel, ou sous gaz inerte. La valeur de la pression est alors comprise entre 50 et 300 mbar, avantageusement de l'ordre de 150 mbar. L'azote est avantageusement utilisé comme gaz inerte.

Sous l'effet conjugué du chauffage, avantageusement par hyperfréquence, et de la pression réduite, l'extraction se déroule jusqu'à épuisement de la substance végétale traitée.

En pratique et de manière avantageuse, l'extraction se déroule à une température inférieure ou égale à 95°C à 150 mbar, et encore plus avantageusement à une température de 55°C sous pression réduite de 150 mbar.

La durée de l'extraction est également maîtrisée, avantageusement inférieure à 1 heure, encore plus avantageusement de l'ordre de 30 minutes, voire de l'ordre de 15 minutes, cette dernière valeur correspondant approximativement au temps de préparation d'une tisane thérapeutique.

Le contrôle de la température comme du temps de réaction pendant la phase d'extraction contribue à limiter l'oxydation des éléments constitutifs de l'extrait végétal en présence.

De préférence, cette étape est réalisée sous agitation.

A l'issue des ces deux étapes, on obtient donc d'une part la matière végétale ayant subi l'extraction et d'autre part la fraction obtenue à l'issue de l'extraction de la matière végétale par le solvant. L'étape suivante correspond à une phase de séchage en présence de ces deux composantes.

Cette étape a pour but essentiel d'entraîner l'évaporation du solvant et est mise en oeuvre jusqu'à séchage complet de l'extrait végétal complet. De manière simultanée, ce séchage permet la fixation des principes actifs extraits sur la matière végétale ayant subi l'extraction.

A l'issue d'un tel procédé, on obtient donc un extrait végétal qui correspond à la matière végétale d'origine dont certains principes actifs ont été extraits au moyen du solvant puis refixés sur celle-ci. Ceci permet de rendre les principes actifs plus disponibles, sans toutefois affecter le « totum » de la matière végétale.

Avantageusement, la matière végétale ne subit pas d'autres traitements que les étapes de macération et d'extraction mises en oeuvre dans le cadre du procédé décrit.

Cette étape est avantageusement réalisée sous pression réduite, ce qui favorise la mise sur support des principes actifs. Les conditions de pression sont telles que décrites précédemment.

De manière remarquable et selon l'invention, les principes actifs sont « sortis » de la matière végétale pendant la phase d'extraction, puis c'est cette même matière végétale qui sert de support d'imprégnation des substances solubles et insolubles pendant la phase de séchage. Il n'y a donc pas d'apport de substrat exogène, et donc pas de dilution des principes actifs.

Avantageusement, l'ensemble de ces étapes, et particulièrement les étapes d'extraction et de séchage, sont réalisées successivement dans une même enceinte hermétique. Une telle enceinte permet de réguler aisément la pression et/ou la température. Le fait de réaliser l'ensemble des étapes du procédé dans une même enceinte permet d'éviter les manipulations et les pertes en principes actifs.

Typiquement, un matériel adapté pour la mise en oeuvre de ce procédé comprend :
- une enceinte à double enveloppe de 100 litres, munie d'une tripale à vitesse variable, par exemple de type Turbosphère TS 100 ;
- une station de vide ;
- un compresseur à air filtré ;
- un générateur d'hyperfréquence de 6000 Watts ;
- un condenseur muni d'une station d'eau frigorifique à 8°C ;
- un groupe chaud d'une puissance de 18 kW.

Dans une étape ultérieure, il est possible de soumettre l'extrait végétal ainsi obtenu à une étape de broyage. Avantageusement, l'extrait peut être granulé pour pouvoir être incorporé dans des sachets à infusion.

Le protocole ainsi défini peut subir des variantes qui sont également couvertes par l'invention.

En particulier, les étapes de macération et d'extraction peuvent être simultanées.

Par ailleurs, ces étapes peuvent être répétées à plusieurs reprises, avantageusement 3, selon le principe de triple extraction. Il est possible d'augmenter la température à chaque répétition. A titre d'exemple, la température à pression atmosphérique peut être de 30°C au premier cycle, puis 60°C et enfin 90°C.

Dans cette optique et de manière avantageuse, après extraction et avant séchage, de la matière végétale brute (non traitée) est ajoutée dans l'extrait, ledit extrait correspondant à la matière végétale initiale et au solvant ayant subi l'étape de contact et d'extraction précédente.

C'est ainsi que dans un mode de réalisation privilégié, le procédé selon l'invention, également appelé « procédé triple macération sous vide à température dirigée », consiste à :
Etape 1 : Macération d'un volume de plante dans l'eau à 30°C, dans une enceinte sous atmosphère protégée (azote).
   A la fin de cette première étape, l'extrait E1 est recueilli.
Etape 2 : Macération d'un nouveau volume de plante dans l'extrait E1 à 60°C, dans une enceinte sous vide.
   On obtient l'extrait E2.
Etape 3 : Macération d'un nouveau volume de plante dans l'extrait E2 à 90°C, dans une enceinte sous vide.
   Les étapes 2 et 3 sont réalisées sous hyperfréquence.
Ensuite, l'ensemble est séché sous vide pour obtenir une poudre de plante imprégnée des extraits précédents.

A l'issue du procédé, l'extrait végétal se présente donc sous forme sèche sur support, le support étant constitué de la cellulose endogène à l'extrait végétal.

L'extrait végétal ainsi obtenu permet, entre autres, de réaliser des tisanes instantanées et donc de conserver le « geste tisane » qui, comme déjà dit, reste la référence en phytothérapie. Dans ce cas, il est conditionné dans des sachets à infusion.

Un tel procédé, et les extraits végétaux qui en résultent, présentent de nombreux avantages, notamment :
- Il s'agit d'un procédé naturel qui peut donc être labellisé « agriculture biologique » ;
- Il peut être mis en oeuvre aussi bien sur du matériel frais que sec ;
- Ce procédé permet de diminuer fortement la contamination microbienne des plantes traitées ;
- Dans la mesure où il est réalisé sous vide partiel ou sous gaz inerte et dans des conditions contrôlées de température, les phénomènes d'oxydation susceptibles d'affecter les principes actifs des plantes sont limités. Ainsi, l'intégrité des actifs contenus dans la matière première végétale est assurée ;
- Dans la mesure où le procédé permet la fixation des actifs préextraits sur l'extrait végétal lui-même, leur intégralité est également assurée. La superposition des HPLC entre une simple infusion et la préparation selon l'invention révèle qu'il n'y a pas d'extraction sélective ;
- Dans le cadre de la triple extraction, ce procédé présente un bon rendement, sans dilution des principes actifs : 3 kilos de plantes permettent d'obtenir 1 kg de la poudre finale. Il faut donc 1,5 g de plante pour préparer 0,5 g de la poudre selon l'invention.

Ce procédé permet donc d'augmenter la biodisponibilité (qualitative et quantitative) des substances issues du végétal au niveau digestif, par préextraction.

L'invention et les avantages qui en découlent ressortiront mieux de l'exemple de réalisation suivant. Cet exemple n'est toutefois aucunement limitatif.

***Préparation de l'extrait végétal selon le procédé de l'invention :*** Obtention d'une tisane traditionnelle.

Dans une enceinte dénommée Turbosphère, on place 10 kg de verveine odorante et 90 kg d'eau, on imprègne sous agitation lente. L'extraction est réalisée sous vide et sous hyperfréquence pendant au moins 15 minutes et à une température équivalente à 90 °C (température traditionnelle d'une tisane). Pendant cette phase d'extraction en phase liquide, si une quantité trop importante de solvant s'évapore et si les plantes ne baignent plus dedans, celui-ci doit être remplacé.

Dans l'étape suivante, la double enveloppe est chauffée pour évaporer le solvant jusqu'à séchage complet de la plante.

On obtient l'équivalent de 10 kg de poudre de verveine odorante. Cette matière est ensuite placée dans des sachets infusions à raison de 2 grammes par sachet.

Une étude comparative est effectuée, versus une infusion de verveine achetée dans le commerce, la préparation est identique à l'infusion traditionnelle, c'est-à-dire un sachet dans une tasse de 150 ml avec infusion pendant 3 minutes.

Les taux de matière sèche ont été comparés dans chaque infusé. La préparation selon l'invention renferme 40% de matière sèche supplémentaire par rapport à l'infusé classique.

## Revendications

1. Procédé de préparation d'un extrait végétal, à partir de matière végétale, pour la réalisation d'une tisane comprenant :
- une étape de contact entre la matière végétale et un solvant ;
- au moins une étape d'extraction en phase liquide des principes actifs, réalisée sous chauffage et sous pression réduite ;
- une étape de séchage de la matière végétale ayant subi au moins l'étape d'extraction en présence de la fraction obtenue à l'issue d'au moins l'étape d'extraction, afin de permettre la fixation des principes actifs sur la matière végétale.

2. Procédé selon la revendication 1, **caractérisé en ce que** la matière végétale est constituée de matière fraîche ou sèche.

3. Procédé selon la revendication 1 ou 2, **caractérisé en ce que** le solvant est un mélange hydro-alcoolique, avantageusement hydro-éthanolique, et encore plus avantageusement un mélange 50/50 V/V.

4. Procédé selon l'une des revendications précédentes, ***caractérisé* en ce que** l'étape d'extraction est réalisée sous hyperfréquence et sous pression réduite, avantageusement sous gaz inerte tel que l'azote.

5. Procédé selon l'une des revendications précédentes, ***caractérisé* en ce que** l'étape d'extraction se déroule à une température inférieure ou égale à 95°C à 150 mbar, et encore plus avantageusement à une température de 55°C sous pression réduite de 150 mbar et **en ce qu'**elle a une durée inférieure à 1 heure, encore plus avantageusement de l'ordre de 15 minutes.

6. Procédé selon l'une des revendications précédentes, ***caractérisé* en ce que** l'étape de séchage est réalisée sous pression réduite.

7. Procédé selon l'une des revendications précédentes, ***caractérisé* en ce que** au moins les étapes d'extraction et de séchage sont réalisées dans une même enceinte hermétique.

8. Procédé selon l'une des revendications précédentes, ***caractérisé* en ce qu'**après l'étape de séchage, l'extrait végétal est soumis à une étape de broyage, et éventuellement de granulation.

9. Procédé selon l'une des revendications précédentes, ***caractérisé* en ce que** l'étape de contact avec le solvant et l'étape d'extraction sont réalisées simultanément.

10. Procédé selon l'une des revendications précédentes, ***caractérisé* en ce que** l'étape d'extraction est répétée plusieurs fois, avantageusement trois fois.

11. Procédé selon la revendication 10, ***caractérisé* en ce que**, entre chaque répétition de l'étape d'extraction, de la matière végétale est ajouté à l'extrait.

12. Procédé selon la revendication 10 ou 11, ***caractérisé* en ce qu'**à chaque répétition, la température d'extraction est augmentée.

## Patentansprüche

1. Verfahren zur Zubereitung eines Pflanzenextrakts auf der Basis eines pflanzlichen Ausgangsmaterials zur Herstellung eines Kräutertees, das folgende Schritte beinhaltet:
- eine Kontaktphase zwischen dem pflanzlichen Ausgangsmaterial und einem Lösungsmittel;
- mindestens eine Extraktionsphase in Flüssigphase der Wirkstoffe, die unter Erhitzung und bei vermindertem Druck erfolgt;
- eine Trockenphase des pflanzlichen Materials, das in Gegenwart der erhaltenen Fraktion mindestens einer Extraktionsphase unterzogen wurde, um die Fixierung der Wirkstoffe auf dem pflanzlichen Material zu ermöglichen.

2. Verfahren gemäß Anspruch 1, **dadurch gekennzeichnet, dass** das pflanzliche Material aus frischem oder getrocknetem pflanzlichen Material besteht.

3. Verfahren gemäß Anspruch 1 oder 2, **gekennzeichnet dadurch, dass** es sich bei dem Lösungsmittel um ein wässrig-alkoholisches Gemisch handelt, vorzugsweise um ein Ethanol-Wasser-Gemisch, wobei ein Mischungsverhältnis von 50/50 v/v optimal ist.

4. Verfahren gemäß einem der vorstehenden Ansprüche, **dadurch *gekennzeichnet,* dass** die Extraktionsphase mit Mikrowelle bei vermindertem Druck erfolgt, vorzugsweise unter einem Inertgas wie Stickstoff.

5. Verfahren gemäß einem der vorstehenden Ansprüche, **dadurch *gekennzeichnet,* dass** die Extraktionsphase bei einer Temperatur unter oder gleich 95°C bei 150 mbar - und vorzugsweise bei einer Temperatur von 55 °C bei einem verminderten Druck von 150 mbar - erfolgt und weniger als 1 Std. dauert, wobei eine Dauer von ca. 15 Min. optimal ist.

6. Verfahren gemäß einem der voranstehenden Ansprüche, **dadurch *gekennzeichnet,* dass** die Trockenphase bei vermindertem Druck erfolgt.

7. Verfahren gemäß einem der vorstehenden Ansprüche, **dadurch *gekennzeichnet,* dass** zumindest die Extraktions- und Trockenphase in demselben hermetischen Gehäuse erfolgen.

8. Verfahren gemäß einem der vorstehenden Ansprüche, **dadurch *gekennzeichnet,* dass** der Pflanzenextrakt nach der Trockenphase zerkleinert und eventuell granuliert wird.

9. Verfahren gemäß einem der vorstehenden Ansprüche, **dadurch *gekennzeichnet,* dass** die Kontaktphase mit dem Lösungsmittel und die Extraktionsphase gleichzeitig erfolgen.

10. Verfahren gemäß einem der vorstehenden Ansprüche, **dadurch *gekennzeichnet,* dass** die Extraktionsphase mehrmals wiederholt wird, vorzugsweise drei Mal.

11. Verfahren gemäß Anspruch 10, **dadurch *gekennzeichnet,* dass** zwischen jeder Wiederholung der Extraktionsphase das pflanzliche Material zum Extrakt hinzugefügt wird.

12. Verfahren gemäß dem Anspruch 10 oder 11, **dadurch *gekennzeichnet,* dass** bei jeder Wiederholung die Extraktionstemperatur erhöht wird.

## Claims

1. Process for preparation of a plant extract from plant material, for the preparation of an infusion including:
- a stage of contact between the plant material and a solvent;
- at least one stage of extraction in liquid phase of the principal active ingredients, performed with heating and under low pressure;
- a stage of drying of the plant material having undergone at least the stage of extraction in the presence of the fraction obtained at the end of at least the extraction stage, in order to allow the fixing of the main active ingredients onto the plant material.

2. Process in accordance with claim 1, **characterized by** the fact that the plant material is composed of fresh or dry material.

3. Process in accordance with claims 1 or 2, **characterized by** the fact that the solvent is a hydro-alcoholic mixture - advantageously hydro-ethanolic and, even more advantageously, a 50/50 V/V mixture.

4. Process in accordance with one of the preceding claims, **characterized by** the fact that the stage of extraction is performed under microwaves and under low pressure - advantageously under an inert gas such as nitrogen.

5. Process in accordance with one of the preceding claims, **characterized by** the fact that the stage of extraction takes place at a temperature of 95°C or less, at 150 mbar and, even more advantageously, at a temperature of 55°C, under low pressure of 150 mbar, and by the fact that it lasts less than 1 hour and, ever more advantageously, around 15 minutes.

6. Process in accordance with one of the preceding claims, **characterized by** the fact that the drying stage is performed under low pressure.

7. Process in accordance with one of the preceding claims, **characterized by** the fact that at least the stages of extraction and drying are performed in the same hermetically-sealed vessel.

8. Process in accordance with one of the preceding claims, **characterized by** the fact that, after the drying stage, the plant extract is subjected to a stage of grinding and, possibly, granulation.

9. Process in accordance with one of the preceding claims, **characterized by** the fact that the stage of contact with the solvent and the extraction stage are performed simultaneously.

10. Process in accordance with one of the preceding claims, **characterized by** the fact that the extraction stage is repeated several times - advantageously three times.

11. Process in accordance with claim 10, **characterized by** the fact that, between each repetition of the extraction stage, plant material is added to the extract.

12. Process in accordance with claims 10 or 11, **characterized by** the fact that, at each repetition, the extraction temperature is increased.
